# EUROPEAN PATENT SPECIFICATION

(11) **EP 0 605 422 B1**
(45) Date of publication and mention of the grant of the patent: **08.03.2000**
(21) Application number: 92906319.6
(22) Date of filing: 23.09.1991
(51) Int. Cl.: A61M 5/32

(54) **SAFETY SYRINGE ASSEMBLY WITH RADIALLY DEFORMABLE BODY**
SICHERHEITSEINRICHTUNG FÜR SPRITZE MIT RADIAL DEFORMIERBAREM KÖRPER
ASSEMBLAGE DE SERINGUE DE SECURITE AVEC CORPS DEFORMABLE RADIALEMENT

(43) Date of publication of application: 13.07.1994
(73) Proprietor: ABBOTT LABORATORIES, Abbott Park, Illinois 60064 (US)
(72) Inventor: HABER, Terry, M. c/o Eastman Kodak Company, Rochester, New York 14650-2201 (US); SMEDLEY, William, H. c/o Eastman Kodak Company, Rochester, New York 14650-2201 (US); LEWIS, John, A c/o Eastman Kodak Company, Rochester, New York 14650-2201 (US); Lewis, John A. c/o Eastman Kodak Company, Rochester, New York 14650-2201 (US)
(74) Representative: Staub, Gabriella, Dr.
(86) International application number: US9106878
(87) International publication number: WO9305834

(56) References cited:
- GB-A- 2 202 747
- US-A- 4 655 751
- US-A- 4 758 230
- US-A- 4 871 355
- US-A- 4 874 383
- US-A- 4 900 311
- US-A- 4 927 417
- US-A- 4 935 016
- US-A- 5 067 945

## Description

### BACKGROUND OF THE INVENTION

The need for preventing inadvertent needle sticks has been recognized for many years. For example, U.S. Patent No. 2,571,653 to Bastien shows a syringe in which the barrel of the syringe is mounted within a protective sheath. The sheath can be placed at different axial positions relative to the barrel, one exposing the needle for use and one covering the needle for safety. Many other safety syringe assemblies have been developed as well (see in particular document GB-A-2202747). However, one of the problems with the prior art safety syringe assemblies is that they generally cannot be used with conventional syringes, are awkward to use and make calibrations and volume graduations difficult to see clearly. Also, existing safety syringe assemblies often are not economical to produce; this is especially true when the syringe structures are intended to be disposable.

### SUMMARY OF THE INVENTION

The present invention is directed to a syringe assembly, particularly useful for use with conventional prefilled pharmaceutical cartridge-needle units, including a hollow body housing the cartridge-needle unit or other syringe structure. The hollow body and the syringe structure include mating positioning elements to permit the syringe structure to be secured at one or more axial positions within the body. The positioning elements are preferably radially extending recesses and extensions. The hollow body typically has at least two internal recesses sized and positioned to engage a radially extending member carried by the syringe structure so that the needle can be partially or totally exposed for use and then withdrawn into the body for safety. The body is configured so that when the user squeezes the body at positions diametrically adjacent the recess, the radially extending member becomes disengaged from the recess due to the deformation of the body. This frees the needle permitting it to be repositioned within the body.

The body is constructed so that the body may be deformed to permit the radially extending member to freely move from the recess to permit the syringe structure and body to move axially relative to one another. This may be achieved by constructing the body with an elliptical cross-sectional shape and forming pairs of the recesses through the walls of the body while constructing the radially extending member as an annular, outwardly extending ring carried by the syringe structure. The radially extending member can be disengaged from the recesses by squeezing the body to deform the body from the generally elliptical shape to a generally circular shape. Preferably, three sets (or four sets to permit one molded body to accommodate both short and long needle lengths) of axially spaced-apart recesses are provided. One recess near the distal end of the body for I.M. (IntraMuscular) injections. An intermediate recess (or two to accommodate both long and short needle cannula) such that the tip of the needle is within the interior of the body may be used for safer disposal. Also, this position may be used for I.V. (IntraVenous) port injection. By properly sizing the opening at the distal end of the body, the body may be placed over the injection valve of an I.V. port, permitting the sharpened point of the needle cannula to pierce the membrane port only after this sharpened point has been shielded from human contact. This permits I.V. port injection with a high degree of safety and efficacy. The recess or recesses closest to the plunger end of the body are used to ensure the needle tip is well within the body for safe disposal of the syringe structures.

One of the primary advantages of the invention is that it permits the needle of a syringe structure to be safely housed within the body in an extremely simple and cost effective manner. In addition, although the invention can be used with conventional syringes, the invention finds particular utility when used with prefilled pharmaceutical cartridge-needle units. In such case, the body can be part of an enclosure unit, the enclosure unit acting as both the protective packaging for the cartridge-needle unit, and also as a part of the operational syringe assembly itself.

Other features and advantages will appear from the following description in which the preferred embodiments have been set forth in detail in conjunction with the accompanying drawings.

### BRIEF DESCRIPTION OF THE DRAWINGS

FIG. 1 is a side cross-sectional view of an enclosure unit made according to the invention;
FIG. 2 is an isometric view of a set of the enclosure units of FIG. 1;
FIG. 3 shows the enclosure unit of FIG. 1 after the adapter has been driven into the body section at the needle end of the body section;
FIG. 4 illustrates a syringe assembly made according to the invention by mounting a cartridge-needle unit mounted within the body section of the enclosure unit of FIG. 3 with the cap at the end section used to plug the end section to maintain the cartridge-needle unit within the enclosure unit and help prevent tampering;
FIG. 5 illustrates the syringe assembly of FIG. 4 after the end unit has been separated from the body section and discarded and the stem section has been separated from the adapter and mounted to the piston at the plunger end of the barrel of the cartridge-needle unit;
FIG. 6 illustrates the syringe assembly of FIG. 5 after the sheath has been removed from the needle and the plunger has been depressed, injecting the contents of the barrel through the needle;
FIG. 7A is a cross-sectional view taken along line 7-7 of FIG. 6 showing the elliptical cross-sectional shape of the body section and the engagement of the adapter ring with the circumferential extending body slots formed in the body section at the needle end of the body section;
FIG. 7B illustrates the syringe assembly of FIG. 7A after the user has deformed the body section by squeezing the body section as indicated to disengage the adapter ring from the body slots to permit the cartridge-needle unit to be moved axially within the body section;
FIG. 8 illustrates the movement of the cartridge-needle unit from the use position of FIG. 6, with the needle exposed, to the safe position of FIG. 8, with the needle fully housed within the body section, and also illustrates how the syringe assembly of FIGS. 6 and 8 could be used to inject a medicine into, or remove a sample from, an injection arm of an IV port while keeping the sharpened tip of the needle in a safe position;
FIG. 9A is an enlarged view taken along line 9A of FIG. 8 illustrating the engagement of a standard adapter ring within a body slot;
FIG. 9B shows an alternative embodiment of the adapter ring of FIG. 9A in which the plunger facing surface of the ring provides a ramp effect to enhance movement of the cartridge-needle unit towards the plunger end of the body section;
FIG. 10 illustrates the syringe assembly of FIG. 6 in which the adapter ring is positioned within the body slots adjacent the plunger end of the body section to accommodate longer needles than used with the embodiment of FIG. 8;
FIGS. 11 and 12a illustrate alternative embodiments of the syringe structure of FIG. 8 adapted for use with different length needles and including different width body slots to accommodate different width adapter rings so the same body section can be used with needles of different lengths and still maintain the proper position of the tip of the needle for IV port use;
FIG. 12b is a partial side view of the syringe structure of FIG. 12a;
FIGS. 13 and 14 illustrate a further alternative embodiment of the invention in which the adapter ring does not extend completely around the adapter to engage the appropriately positioned body slot for a short needle version of the cartridge needle unit;
FIGS. 15 and 16 show the embodiment of FIGS. 13 and 14 with a long needle version of the cartridge-needle unit and with an adapter ring positioned to the opposite side of the body section as in the embodiment of FIG. 13, so that the adapter ring of the embodiments of FIGS. 13 and 15 engage the appropriately intermediate position body slots located at different axial positions along the body section according to the lengths of the needles;
FIG. 17 illustrates a further embodiment of the invention in which the outer surface of the body section is normally cylindrical with the inner surface having internal flanges which engage a circumferential slot formed in an adapter mounted to the hub of the cartridge-needle unit;
FIG. 18A is a cross-sectional view of the syringe assembly of FIG. 17 taken along line 18-18 illustrating the engagement of the flanges into the annular slot;
FIG. 18B illustrates the syringe assembly of FIG. 18A after the body section has been deformed causing the flanges to disengage from the annular slot to permit the cartridge-needle unit to move axially within the body section;
FIG. 19 illustrates an alternative embodiment of the invention in which the syringe assembly of FIG. 4 has been modified to eliminate the need for a separate adapter by using a cartridge-needle unit in which the hub of the cartridge-needle unit is modified to include its own adapter ring;
FIG. 20 shows a further embodiment of the invention for use with smaller sized cartridge-needle units; and
FIG. 21 is a cross-sectional view taken along line 21-21 of FIG. 20.

### DETAILED DESCRIPTION OF THE PREFERRED EMBODIMENT

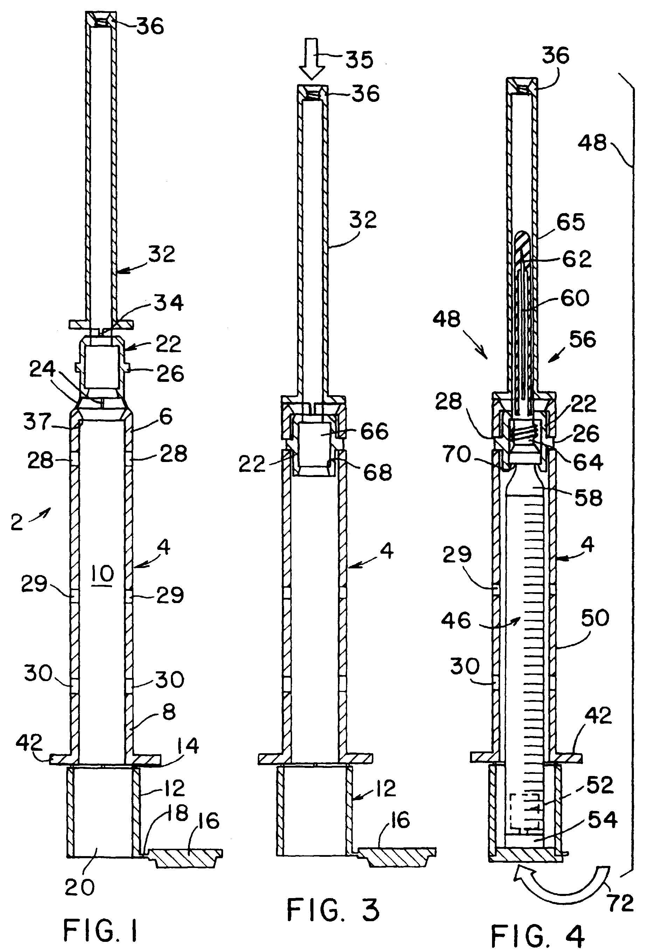

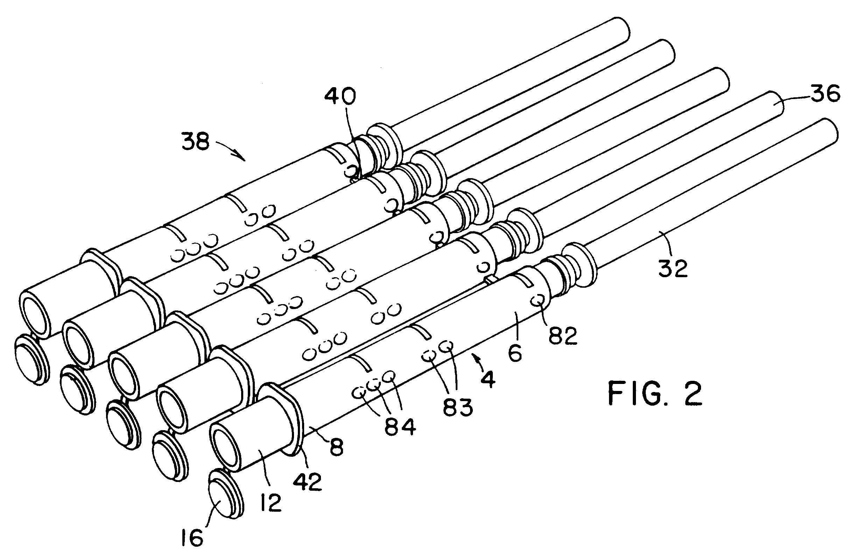

FIG. 1 illustrates a one-piece molded enclosure unit 2, preferably made from clear polypropylene or polyethylene. Enclosure unit 2 includes elongate, substantially hollow body section 4 having a needle end 6, a plunger end 8 and defining an interior 10 therein. Unit 2 also includes an end section 12 frangibly connected to plunger end 8 at frangible connections 14. End section 12 includes a cap 16 connected to the remainder of end section 12 by an integral hinge 18 to permit the end 20 of end section 12 to be oriented and sealed as suggested in FIG. 4.

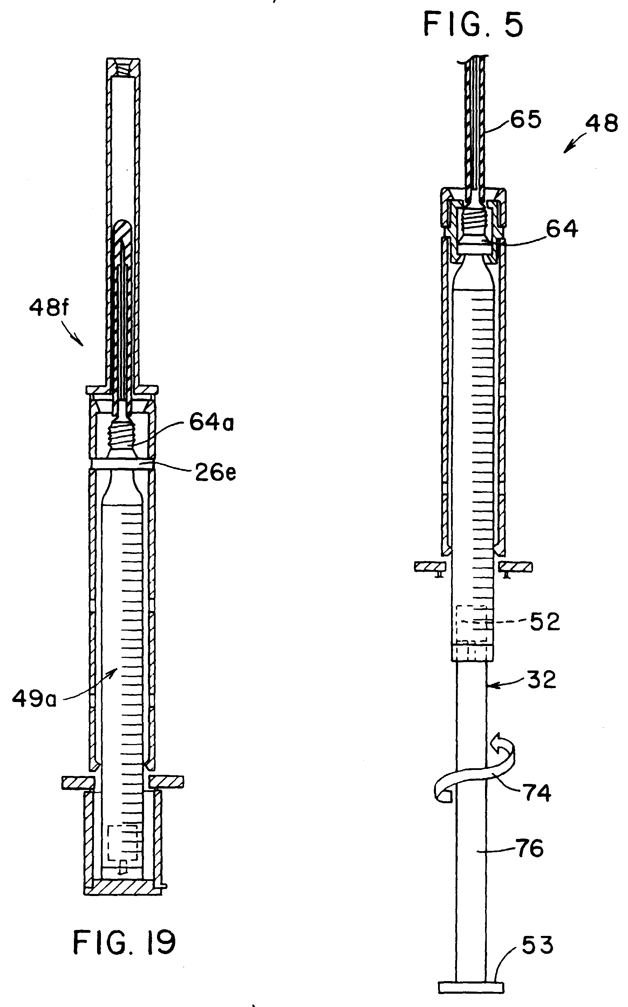

Enclosure unit 2 also includes an adapter 22 extending from needle end 6 and attached thereto by frangible connections 24. Adapter 22 has a circumferentially extending adapter ring 26 sized to engage specially positioned pairs of body slots 28, 29, 30 formed in body section 4. The method and purpose for doing so will be discussed below. Finally, enclosure unit 2 also includes a hollow stem section 32 frangibly attached to adapter 22 by frangible connections 34. Stem section 32 is hollow to accommodate a sheathed (typically sterile) needle (see FIG. 4) and also has a threaded tip 36 to permit stem section 32, once removed from adapter 22, to be secured to a piston to create a plunger as suggested in FIG. 5. Adapter 22 is moved from the position of Fig. 1 to that of Fig. 3 by forcing stem section 32 in the direction of arrow 35, thus severing frangible connections 24, until ring 26 engages slots 28. Body section 4 includes a shoulder 37 at needle end 6. Shoulder 37 helps to keep adapter 22 from inadvertently passing back out of interior 10. Shoulder 37 may be a continuous shoulder, or it may be formed as diametrically opposed segments.

FIG. 2 illustrates a set 38 of enclosure units 2 molded as a one-piece item and secured to one another through frangible connections 40 at needle end 6 of body section 4 and by frangible connections (not shown) extending between the finger grips 42 at plunger ends 8. As discussed in the above-referenced application, set 38 of enclosure units 2 acts as both the protective shipping and storage container for cartridge-needle units 46, shown in Fig. 4, as well as providing other functions as discussed below.

FIG. 4 illustrates enclosure unit 2 with a conventional cartridge-needle unit 46, such as one made by Wyeth-Ayerst Laboratories of Radnor, Pennsylvania, under the trademark TUBEX, mounted therein to create a syringe assembly 48 in its as-shipped condition. Cartridge-needle unit 46 is of the type including a barrel 50 containing a flowable material, typically a liquid, and having a piston 52, typically initially at the plunger end 54 of barrel 50. A needle assembly 56 is mounted to the needle end 58 of barrel 50. Needle assembly 56 includes a needle 60, having a sharpened tip 62, and a hub 64 used to mount needle 62 barrel 50. An elastomeric sheath 65 is used to cover needle 60 and seal tip 62. Hub 64 is secured within the interior 66 of adapter 22 through the engagement of a lip 68 (see FIG. 3) with the plunger facing edge 70 of hub 64. As suggested by arrow 72, once cartridge-needle unit 46 is mounted within enclosure unit 2, cap 16 is pivoted to close end 20 and is sealed in place, such as with an irreversible mechanical lock which is also tamper-resistent and tamper-evident, an adhesive or with heat or ultrasonic welding techniques. The thickness of cap 16 is sufficient to resist tampering with the contents of barrel 50 while syringe assembly 48 is in its as-shipped condition of FIG. 4. To further inhibit tampering, an internal metal shield could be used.

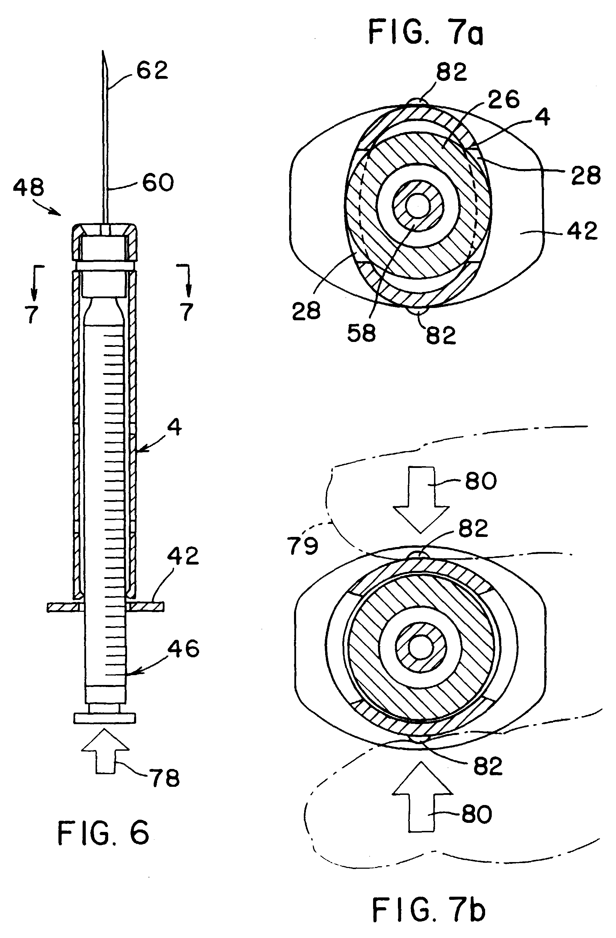

To use syringe assembly 48, stem section 32 is fractured from adapter 22, typically with a twisting action, and tip 36 is secured to piston 52, typically with a threading action as suggested by arrow 74 in FIG. 5. Next, sheath 65 is removed and the injection is given by driving plunger 76, made up of stem 32, piston 52 and thumb plate 53, as shown by arrow 78 of FIG. 6.

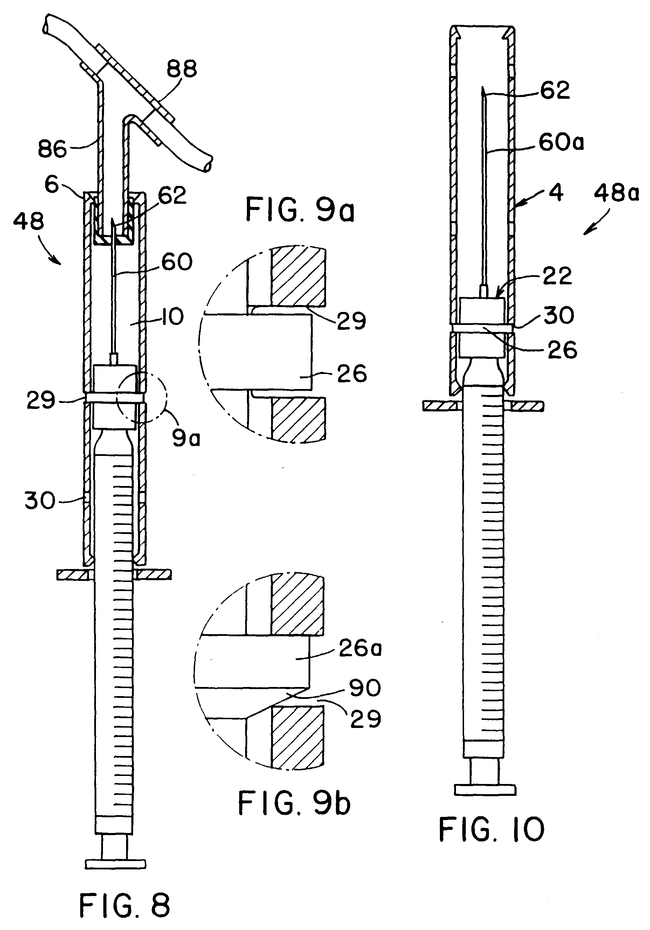

The present invention permits needle 60 to be withdrawn back into body section 4 in a simple, straightforward manner. As shown in FIG. 7A, body section 4 has an elliptical cross-sectional shape with body slots 28 positioned through the walls of body 4. This permits the outer circumference of adapter ring 26 to enter body slots 28 so long as body section 4 is in its normal, typically undeformed condition of FIG. 7A. To disengage adapter ring 26 from body slots 28 thereby permitting its axial movement, the user 79 squeezes body 4 as suggested by arrows 80 in FIG. 7B opposite body slot 28. The appropriate place to squeeze body section 4 is further aided by the provision of three different sets of tactile bumps 82, 83, 84, shown in FIG. 2, opposite body slots 28, 29 and 30. Once body section 4 has been sufficiently deformed to release adapter ring 26, cartridge-needle unit 46 can be moved axially relative to body section 4 from the I.M. injection position of FIG. 6 to the I.V. injection position of FIG. 8, at which adapter ring 26 engages body slots 29. As can be seen in FIG. 8, body slots 29 are positioned so that tip 62 of needle 60 is within interior 10 of body section 4, but near needle end 6. Doing so permits syringe assembly 48 to be easily and safely used to inject a liquid into or withdraw a liquid from a membrane valve 86 of a conventional I.V. port 88 as shown in FIG. 8. The opening at needle end 6 is sized to properly guide injector arm 86 while eliminating any substantial possibility of an inadvertent needle stick during such a procedure.

FIG. 9A illustrates the engagement of adapter ring 26 within body slot 29. At FIG. 9B a modified adapter ring 26a is shown with a tapered plunger-facing surface 90. The use of surface 90 keeps adapter ring 26 snug within body slot 29 and also provides a ramp effect to make movement of adapter ring 26a towards plunger end 8 of body section 4 easier.

FIG. 10 illustrates a syringe assembly 48a identical to syringe assembly 48 but with a needle 60a being longer than needle 60. To properly position tip 62 relative to needle end 6, adapter 22 is positioned along body section 4 until adapter ring 26 engages body slots 30 rather than body slots 29.

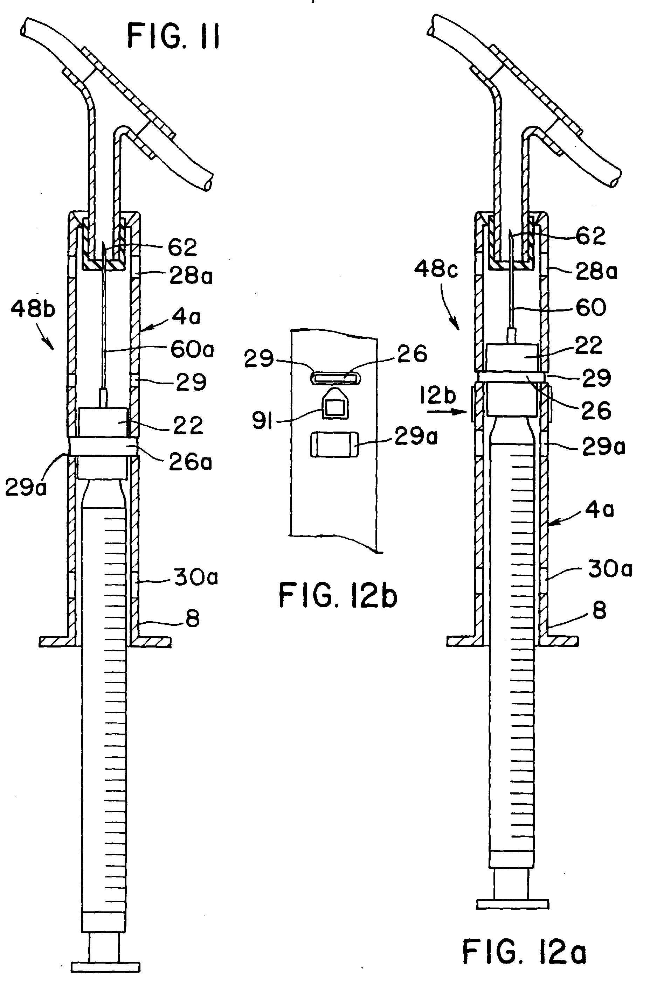

FIGS. 12a and 11 illustrate further embodiments providing for the proper positioning of tips 62 of needles 60, 60a (the short and long needle versions, respectively). Short needle 60 is used with a conventional width adapter ring 26 as in FIG. 8. However, with syringe assembly 48b, shown in FIG. 11, extra width adapter ring 26b is used. Adapter ring 26b will engage body slots 28a, 29a and 30a but will not engage narrow width body slots 29. However, normal width adapter ring 26 can engage all of the body slots 28a, 29, 29a and 30a. Therefor, adapter ring 26b is repositioned from body slots 28a, past body slots 29 and into engagement with body slots 29a so to properly position tip 62. With syringe assembly 48c, shown in FIG. 12, adapter ring 26 is initially positioned within body slots 29a (although somewhat loosely). Movement of adapter ring 26 from body slot 28a towards plunger end 8 allows adapter ring 26 to engage body slots 29 and thus properly position tip 62 in this case as well. FIG. 12b shows the use of an I.V. drip visual indicator 91 between body slots 29, 29a to show the user which body slot is to be used for use with an I.V. port 88. Other visual indicators or markings could be used adjacent the body slots used for I.M. injections and safe disposal.

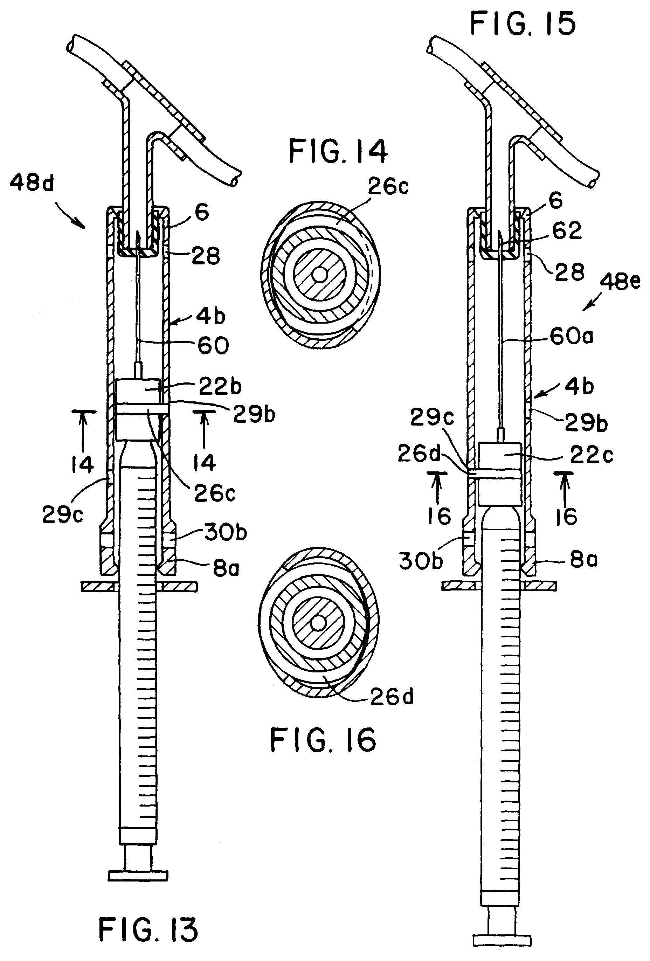

FIGS. 13-16 illustrate another method by which different length needles can be accommodated, and also demonstrate that adapter ring 26 need not be a full circle. Syringe assembly 48d, illustrated in FIG. 13, uses a short length needle 60 while syringe assembly 48e uses long length needle 60a while both use a body section 4b. Body section 4b includes a pair of body slots 28, a single body slot 29b, a single body slot 29c and a pair of body slots 30b formed in a plunger end 8a. As can be seen in FIG. 14, adapter ring 26c is generally C-shaped and is sized to substantially impede or prevent rotation of adapter 22b within interior 10 of body section 4b when engaged within the body slot 29b. The axial and rotary position of body slot 29b is chosen to properly position tip 62 of needle 60. Similarly, with reference to FIGS. 15 and 16, adapter 22c has its adapter ring 26d positioned 180° from adapter ring 26c of FIG. 14 so that adapter ring 26d engages body slot 29c rather than slot 29b so tip 62 of needle 60a is also properly positioned relative to needle end 6 of body section 4b.

In both embodiments of FIGS. 13 and 15, plunger end 8a is thickened or strengthened. This helps to rigidify body section 4b at body slots 30a so that once adapter rings 26c, 26d enter body slots 30a, it will be quite difficult to squeeze plunger end 8a of body section 4b with sufficient force to disengage the adapter ring from the body slot.

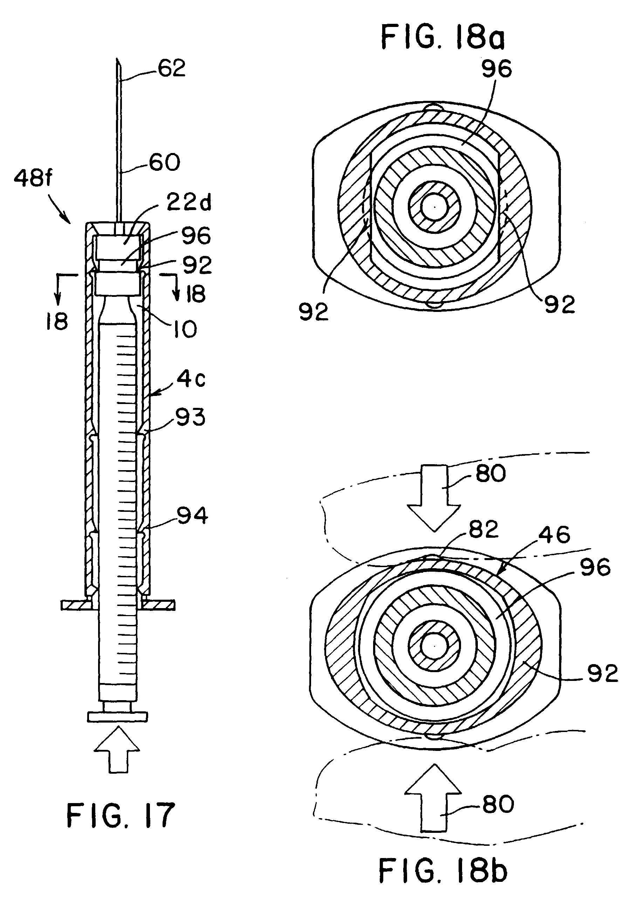

FIGS. 17, 18A and 18B illustrates a situation in which the body slots 28, 29 and 30 are replaced by flanges 92, 93, 94 which engage an annular slot 96 formed in adapter 22d. The disengagement of flanges 92 from slot 96 occurs in the same manner as discussed with reference to FIGS. 7A and 7B. That is, the user squeezes body section 4c as shown by arrows 80 to cause the disengagement of flanges 92 from slot 96. This permits the relative axial movement of adapter 22d within body section 4c so to reposition needle 60 within the interior 10 of the body section.

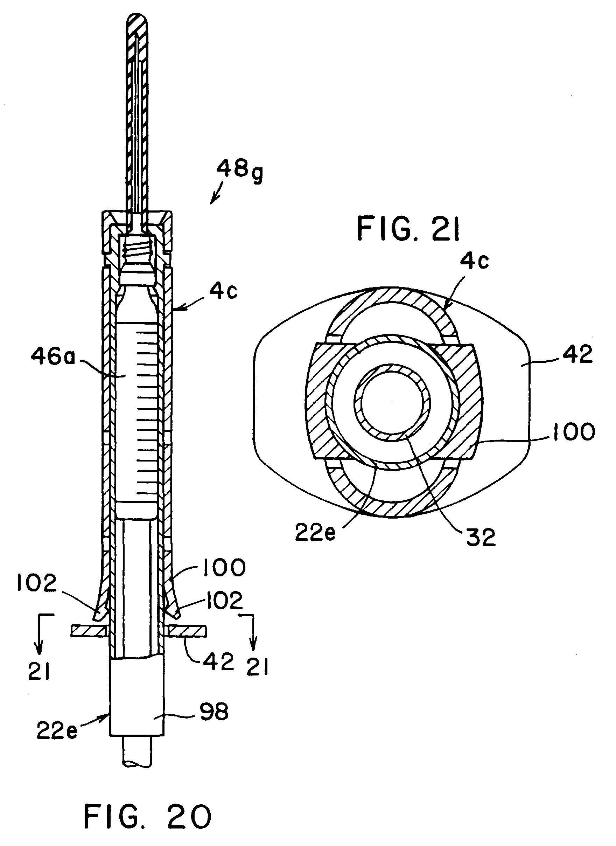

FIG. 19 illustrates a still further alternative embodiment of the syringe assembly 48 of FIG. 4. Syringe assembly 48f is similar to syringe assembly 48 except that adapter 22 is replaced by providing an adapter ring 26e as an integral part of hub 64a. This eliminates the need for adapter 22, but likely requires modification of a conventional cartridge-needle unit.

Turning now to FIGS. 20 and 21, a syringe assembly 48g is shown adapted for use with a smaller sized cartridge-needle unit 46a. One distinction between syringe assembly 48g and syringe assembly 48 is the use of an extended length adapter 22e, a portion 98 of which extends past finger grips 42 to permit user 79 to grasp portion 98 when placing the assembly into an I.V. injection or a safe disposal condition.

Body section 4c of syringe assembly 48g includes a pair of inwardly biased fingers 100 having protrusions 102 shaped to conform to the outer surface of portion 98 of adapter 22e. This helps to center adapter 22e, and cartridge-needle unit 46a therewith, within interior 10 of body section 4c. Fingers 100 could also be used to center and stabilize cartridge-needle unit 46 in the other embodiments.

Other modifications and variations can be made to disclosed embodiments without departing from the subject of the invention as defined in the following claims. For example, the invention is particularly adapted for use with a cartridge-needle unit. However, the invention is also adaptable for use with syringes and can be used with separately packaged cartridges and cartridge-needle units as well. While most of the disclosed embodiments use an elliptically shaped body section, the body section could, however, be another shape, such as generally triangular, as well. The deformation of the body section could also be created by twisting or rotating the adapter ring (or other radially extending member) to cause the radially extending member to disengage from a body slot. However, doing so would require the appropriate rotary manipulation of the pieces in addition to axial manipulation. While enclosure unit 2 is, in the preferred embodiment, a one-piece molded structure, it may be desireable to make it from two or more pieces. For example, it may be desireable to make adapter 22 and cap 16 as separate pieces. Also, it may be desired to mount adapter 22 to hub 64 prior to positioning the cartridge-needle unit within the body section.

## Claims

1. Syringe assembly comprising:
a syringe structure;
the syringe structure including a first positioning element;
a hollow body (4) housing at least a portion of the syringe structure; and
the body (4) including an inner surface having a second positioning element formed thereat, the first and second positioning elements sized for mating engagement when aligned so to prevent relative axial movement of the body and the syringe structure;
characterized in that one of the first and second positioning elements includes a radially extending circumferential member (26) extending around a substantial portion of the circumference thereof and the other of the first and second positioning elements includes at least one recess (28, 29,30) sized to accept the radially extending member (26) regardless of the relative rotary orientation of the syringe structure and the body;
the body (4) including means for permitting at least a part of the body to be deformed radially when the radially extending circumferential member (26) is to be removed from said at least one recess (28,29,30) to allow relative axial movement of the body and the syringe structure.

2. Assembly according to claim 1 wherein the first positioning element includes said radially extending circumferential member (26).

3. Assembly according to claim 1 wherein the second positioning element includes said radially extending circumferential member (26).

4. Assembly according to any of claims 1 to 3 wherein the syringe structure includes a cartridge-needle unit (46) having a barrel (50), a piston (52) mounted within the barrel, and a needle assembly (56) mounted to the barrel.

5. Assembly according to claim 4 wherein the syringe structure includes an adapter (22) mounted to the cartridge-needle unit, the adapter including the first positioning member.

6. Assembly according to any of claims 1 to 5 wherein the radially extending member includes an annular ring (26).

## Patentansprüche

1. Ein Spritzenaufbau, der folgendes umfaßt:
eine Spritzenvorrichtung;
wobei die Spritzenvorrichtung ein erstes Positionierungselement einschließt:
einen hohlen Körper (4), der mindestens einen Abschnitt der Spritzenvorrichtung unterbringt; und
wobei der Körper (4) eine Innenfläche einschließt, die über ein daran ausgebildetes zweites Positionierungselement verfügt, wobei das erste und zweite Positionierungselement für einen zusammenpassenden Eingriff dimensioniert sind, sobald sie ausgerichtet sind, um so die relative axiale Bewegung des Körpers und der Spritzenvorrichtung zu verhindern;
dadurch gekennzeichnet, daß eines der ersten und zweiten Positionierungselemente ein sich radial erstreckendes Umfangsglied (26) einschließt, das sich um einen wesentlichen Abschnitt des Umfangs davon erstreckt, und das andere der ersten und zweiten Positionierungselemente mindestens einen Einschnitt (28, 29, 30) einschließt, der dimensioniert ist, um unabhängig von der relativen Drehausrichtung der Spritzenvorrichtung und des Körpers das sich radial erstreckende Glied (26) aufzunehmen;
wobei der Körper (4) ein Mittel einschließt, um mindestens einem Teil des Körpers zu erlauben, radial verformt zu werden, wenn das sich radial erstreckende Umfangsglied (26) aus dem mindestens einen Einschnitt (28, 29, 30) entfernt werden soll, um die relative axiale Bewegung des Körpers und der Spritzenvorrichtung zu gestatten.

2. Der Aufbau nach Anspruch 1, worin das erste Positionierungselement das sich radial erstreckende Umfangsglied (26) einschließt.

3. Der Aufbau nach Anspruch 1, worin das zweite Positionierungselement das sich radial erstreckendes Umfangsglied (26) einschließt.

4. Der Aufbau nach irgendeinem der Ansprüche 1 bis 3, worin die Spritzenvorrichtung eine Patronennadeleinheit (46) einschließt, die über ein Kolbenrohr (50), einen innerhalb, des Kolbenrohrs angebrachten Kolben (52) und einen am Kolbenrohr angebrachten Nadelaufbau (56) verfügt.

5. Der Aufbau nach Anspruch 4, worin die Spritzenvorrichtung einen an der Patronennadeleinheit angebrachten Adapter (22) einschließt, wobei der Adapter das erste Positionierungselement einschließt.

6. Der Aufbau nach irgendeinem der Ansprüche 1 bis 5, worin das sich radial erstreckende Glied einen ringförmigen Ring (26) einschließt.

## Revendications

1. Ensemble formant seringue, comportant:
une structure de seringue;
la structure de seringue (4) incluant un premier élément de positionnement;
un corps creux (4) logeant au moins une partie de la structure de seringue; et
le corps (4) incluant une surface intérieure présentant un second élément de positionnement qui y est formé, le premier et le second éléments de positionnement étant dimensionnés pour s'associer en venant en prise lorsqu'ils sont alignés de façon à empêcher un mouvement axial relatif du corps et de la structure de seringue.
caractérisé par le fait que l'un des deux, du premier et du second éléments de positionnement, comporte un élément circonférentiel (26) s'étendant radialement et s'étendant autour d'une portion substantielle de sa circonférence, et que l'autre des deux éléments de positionnement inclut au moins un évidement (28, 29, 30) dimensionné pour recevoir l'élément (26) s'étendant radialement, quelle que soit l'orientation angulaire relative de la structure de seringue et du corps;
le corps (4) incluant des moyens pour permettre de déformer radialement au moins une partie du corps lorsqu'il faut sortir l'élément circonférentiel (26) s'étendant radialement hors dudit évidement (28, 29, 30), dont il y a au moins un, pour permettre un mouvement axial relatif du corps et de la structure de seringue.

2. Ensemble selon la revendication 1, dans lequel c'est le premier élément de positionnement qui inclut ledit élément circonférentiel (26) s'étendant radialement.

3. Ensemble selon la revendication 1, dans lequel c'est le second élément de positionnement qui inclut ledit élément circonférentiel (26) s'étendant radialement.

4. Ensemble selon l'une quelconque des revendications 1 à 3, dans lequel la structure de seringue inclut un sous-ensemble cartouche-aiguille (46) comportant un cylindre (50), un piston (52) monté dans le cylindre, et un ensemble formant aiguille (56) monté sur le cylindre.

5. Ensemble selon la revendication 4, dans lequel la structure de seringue inclut un adaptateur (22) monté sur le sous-ensemble cartouche-aiguille, l'adaptateur incluant le premier élément de positionnement.

6. Ensemble selon l'une quelconque des revendications 1 à 5, dans lequel, l'élément s'étendant radialement inclut une bague annulaire (26).
